# EUROPEAN PATENT SPECIFICATION

(11) **EP 4 229 172 B1**
(45) Date of publication and mention of the grant of the patent: **03.12.2025**
(21) Application number: 21790817.7
(22) Date of filing: 07.10.2021
(51) Int. Cl.: C12M 1/00, C12M 1/12, B01D 29/05, B01D 29/90

(54) **INLINE FILTER CAP WITH MODULATED FLOWPATH**
INLINE-FILTERKAPPE MIT MODULIERTEM STRÖMUNGSWEG
CAPUCHON DE FILTRE EN LIGNE À TRAJET D'ÉCOULEMENT MODULÉ

(30) Priority: 13.10.2020 US 202063090868 P
(43) Date of publication of application: 23.08.2023
(73) Proprietor: Cytiva Sweden AB, 751 84 Uppsala (SE)
(72) Inventor: ALRIKSSON, Johan, 75184 Uppsala (SE); HERNANDEZ VERA, Rodrigo, 75184 Uppsala (SE); LEVIN, Ori, 412 63 Goteborg (SE); LINDELL, Ida, 412 63 Goteborg (SE)
(74) Representative: Bedford, Grant Richard
(86) International application number: PCT/EP2021/077687
(87) International publication number: WO 2022/078865

(56) References cited:
- WO-A1-96/33770
- DE-A1- 3 731 864
- US-A- 4 159 954
- US-A- 4 229 306
- US-A- 6 030 539
- US-A1- 2004 149 635
- US-A1- 2018 207 555

## Description

### Background

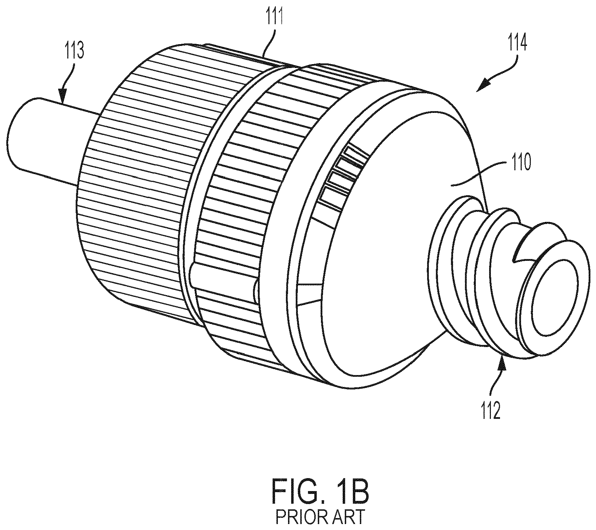

Several designs for in-line filters have been used for years in bioprocessing and related technologies. One known filter cap is the Whatman^{™} Swin-Lok filter holder, which was designed for microfiltration of small volumes of liquids using positive pressure. These holders are used for aseptic sampling of liquids or gases in the laboratory or when samples must be collected and processed on site. A variety of membrane types, including Nuclepore^{™} and Cyclopore^{™} track-etch, cellulosic as well as glass fiber filters can be used in this holder depending upon the application. The filter includes an assembly ring 101, a cap 102, an upper support grid 103, a flat gasket 104, a membrane 105, an O-ring 106, a lower support grid 107 and a base 108. Another type of in-line filter known as a Swinnex filter holder as shown in Fig. 1B. The Swinnex filter 114 has an inlet 112 adapted to allow connection of a syringe, a conical inlet 110 a base 111 and an outlet 113. Theses filters result in dead-end filtration, which can often suffer from clogging due to buildup collecting on the filter surface. Documents US4229306 A and US4159954 A disclose in-line filter caps having flow guiding elements for dispersing the flow radially outward over a filtering surface.

The present inventors have noted a problem with in-line filters in that they experience clogging at the filter interface. With respect to single use sterile products, the need to change filters in process is particularly disadvantageous. These and related problems become more pronounced when the retentate before the filter includes particulate matter such as cellular debris.

### Summary of the Invention

The present invention, as defined by the appended claims, is thus provided.

The filter caps according to the present invention are thus defined by claim 1.

The flow conduit includes a vortex feature configured to provide vortex flow within the filter cap, wherein the vortex feature may be a helical structure. The filter cap may include a recirculating structure, wherein the recirculating structure is a channel located radially outward from the helical structure. The flow conduit may include a dual cone structure for imparting a cross-flow within the filter cap. In the case of the dual cone structure, a recirculation channel may be provided in a central region of the filter cap. Cross-flow increases the flow across the surface of the membrane relative to flow through the membrane. The flow conduit may include a recirculating conduit or a flow restriction feature (e.g., a venturi contraction), or both a recirculating conduit and a flow restriction feature. The filter cap may include a spiral-shaped guide vanes to increase cross-membrane flow.

The filter cap may be manufacturing using at least one additive manufacturing step. The additive manufacturing may include selective laser sintering (SLS), stereolithography (SLA), digital light projection (DLP), continuous liquid interface programming (CLIP), binder jetting, selective hot sintering (SHS), fused deposition modeling (FDM), direct metal laser sintering (DMLS) or directed energy deposition (DED).

### Brief Description of the Drawings

Fig. 1A shows a conventional Whatman^{™} Swin-Lok filter cap.
Fig. 1B shows a conventional "Sweeney" filter cap.
Fig. 2A shows a perspective view of a filter cap with cross section according to an example described herein.
Fig. 2B shows the filter cap in a filter housing with a membrane according to an example described herein.
Fig. 2C shows the fluid domain used for simulation of flow within the filter cap of Fig. 2A.
Fig. 2D shows the simulated effect on flow rate and turbulence on recirculation for the filter cap of Fig. 2A.
Fig. 2E shows the static pressure as a function of the current flowrate in the filter cap of Fig. 2A.
Fig. 2F shows the simulated flow across the membrane surface for the filter cap of Fig. 2A.
Fig. 2G shows recirculation versus flow rate for the filter caps of Fig. 2A (vortex top) and Fig. 6A (dual cone).
Fig. 2H shows Percentile recirculation versus flow rate for the filter caps of Fig. 2A (vortex top) and Fig. 6A (dual cone).
Fig. 3A shows a depiction the interface of an additive manufacturing technique that may be employed to make the filter cap according to one example described herein.
Fig. 3B shows a device for conducting the additive manufacturing technique shown in Fig. 3A according to one example described herein.
Fig. 4 shows a perspective view of a filter cap with cross section according to an example described herein.
Fig. 5A shows a perspective view of a filter cap with cross section according to an example described herein.
Fig. 5B shows the flow vectors for the dual cone filter cap shown in Fig. 5A.
Fig. 5C shows the flow vectors over the filter surface for the dual cone filter cap shown in Fig. 5A.
Fig. 6A shows a perspective view of a filter cap with cross section according to an example described herein.
Fig. 6B shows the simulated effect on flow rate and turbulence on recirculation for the filter cap of Fig. 6A.
Fig. 6C shows the simulated flow velocity in the filter cap of Fig. 6A.
Fig. 6D shows the simulated flow across the membrane surface for the filter cap of Fig. 6A.
Fig. 7A shows a perspective view of a filter cap with cross section according to an example described herein.
Fig. 7B shows the flow vectors for the dual cone AB filter cap shown in Fig. 7A.
Fig. 7C shows the flow vectors over the filter surface for the dual cone AB filter cap shown in Fig. 7A.

### Detailed Description

Various implementations and details are described with reference to filter housings useful in, for example, inline filter assemblies where the filter cap holds a filter membrane against a base, and fluid passes from the filter cap through the filter and out the base. The filter housing includes a conical shape and a flow path configured to modify the flow of the fluid through the inline filter assembly. The inventors have perceived a need for filter housings that allow for controlled fluid flow over a filter membrane within the filter housing. This fluid flow may impart a component of tangential flow or cross flow over the surface of a filter membrane in use. The fluid flow may also involve recirculation of fluid within the cap. The flow regime within the cap may be laminar or turbulent, and that flow regime may be controlled by changing the flow rate, pressure drop, and/or the geometry of the internal flow path of the filter housing. In some cases, transition to turbulent flow imparts a recirculation flow within the filter cap. The filter cap can achieve recirculation in a passive system, e.g., by controlling the liquid flow rate into the filter cap without the need for added pumps or other external energy sources.

The term "conical" as used herein denotes a structure that increases proceeds from a conduit shape at its narrow end and spreads fluid over the surface of a membrane at the large end. The term conical is meant to apply to the inner structure that is configured to distribute fluid over the surface of a filter membrane, and can be independent of the outer shape of the filter housing. For example, the outer surface of a filter cap may be cylindrical yet include a conical interior conduit. The term "conical" describes a general shape and does not impart a mathematically precise cone-shaped geometry. Rather, the smooth transition from a narrower conduit to the membrane is desired to reduce the pressure drop associated with the widening of the fluid flow path relative to a more stepwise increase in size.

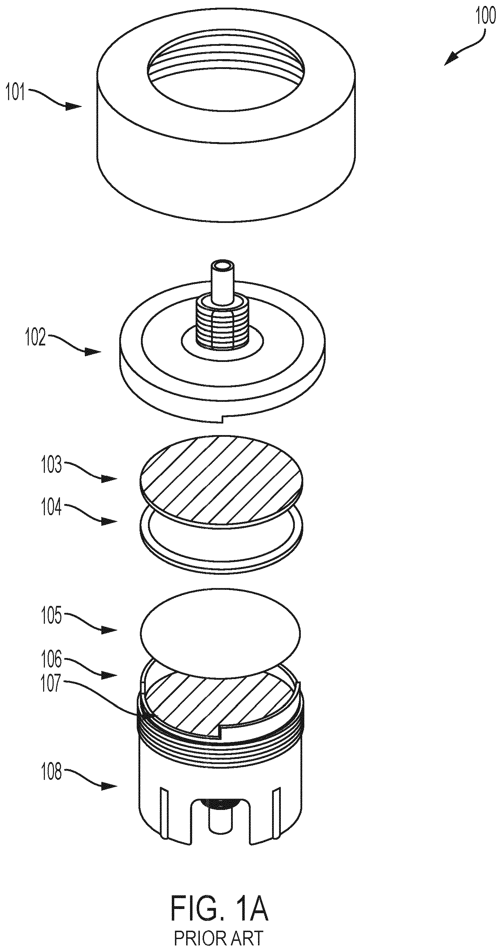

The inventors have contemplated several filter structures for the cap of a filter housing assembly that may be utilized to impart desired flow characteristics. In many cases, the structures utilize a geometry that requires additive manufacturing. For example, the internal geometry may include a structural configuration that has no single "pull plane" from which an injected molded feature may be released from the mold. Earlier filter housing assemblies and filter caps shown above with reference to Figs. 1A-1B include a symmetrical structure capable of construction using traditional injection molding techniques. In many cases, the structural complexity of the filter housing of the examples described herein would prevent the component from being released from a mold, and would therefore preclude manufacture using injection molding techniques.

Several techniques for additive manufacturing, known also as 3D printing, have enabled design of articles with a level of complex internal geometry unavailable years ago. These techniques generally involve building the article layer-by-layer in an additive process. Several examples of additive manufacturing techniques for plastic parts for our target market are selective laser sintering (SLS), stereolithography (SLA), digital light projection (DLP), continuous liquid interface programming (CLIP), binder jetting, selective hot sintering (SHS), fused deposition modeling (FDM). Some of these processes normally require post-processing, such as cleaning with a solvent that can remove powder or cure the polymer into the finished article. In the case of a metal article, other known techniques such as direct metal laser sintering (DMLS) or directed energy deposition (DED) may be used. However, it is contemplated that most filter housing applications would use plastic additive manufacturing techniques. In some cases, the additive technique may include construction of a sacrificial mold that can be printed of a material that can be removed by dissolution after using the sacrificial mold to shape the filter housing. While one additive technique may provide advantages over another additive technique, the filter housings described herein may be made by any additive manufacturing technique or combination of additive techniques.

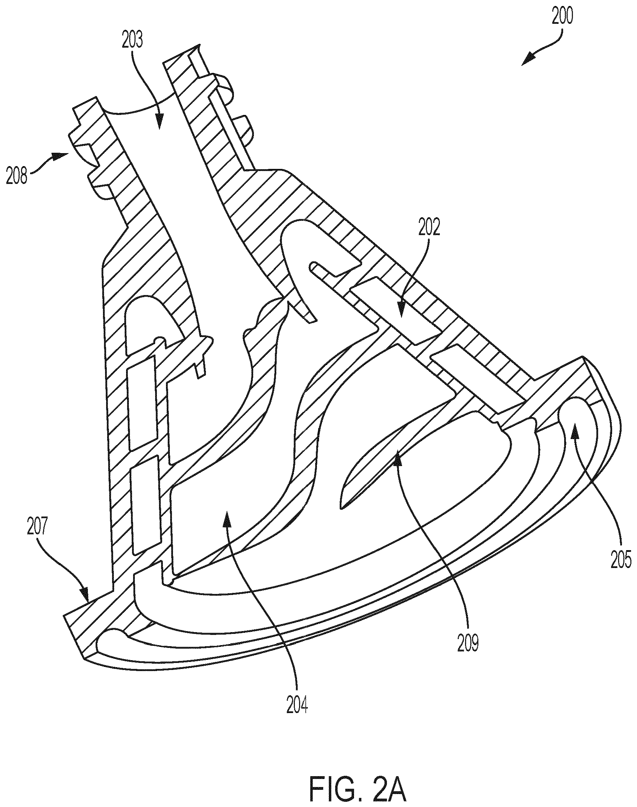

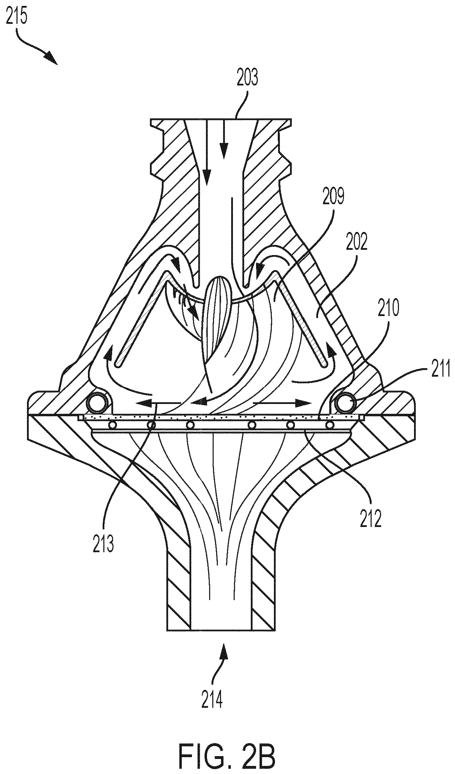

Fig. 2A shows a filter cap 200 to be used with a filter housing assembly according to an example described herein. Fig. 2B shows a filter housing 215 with the filter cap 200 secured to a filter base 214. The filter cap 200 is configured to be fitted onto a filter base 214 with a porous membrane 210 held between the base 214 and the filter cap 200. The filter cap 200 includes a groove 205 that may be adapted to accept an O-ring 211. The filter may have an edge 207 that can be clamped against the base 214 during operation in a conventional manner, using a threaded ring (not shown) that interfaces with a threaded portion of the base (not shown). The filter cap 200 includes an inlet 203. The outer side of the filter cap 200 inlet includes grooves 208 adapted to interface with an inlet tube or syringe. The filter cap 200 includes an internal passage 204 and internal ridges 209 that can create a desired flow under certain flow conditions. The filter cap 200 includes internal channels 202. Under certain flow conditions, including turbulent flow, the internal channels 202 allow for recirculation of flow within the filter housing.

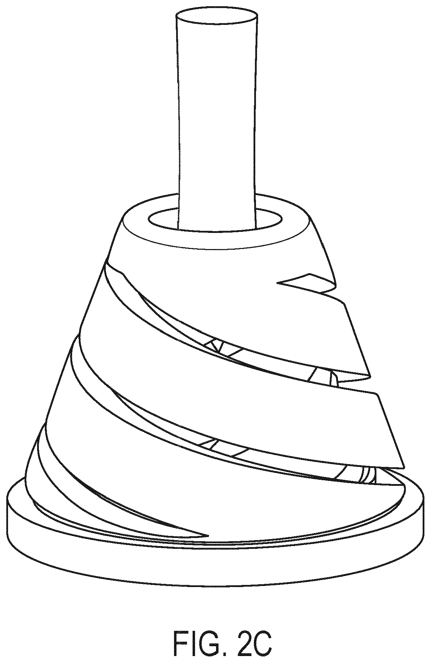

The flow within filter housing 215 using filter cap 200 was simulated at different flow rates. Fig. 2C shows the fluid volume from the computational mesh. The fluid volume was extracted for each design for the geometry in question. Small modifications were made to the geometries to avoid bad cells in the computational mesh. The modifications were considered small enough to have not impacted the flow field. The inlet and outlet were given constant pressure, and the flow rate was adjusted by modifying the resistance over the porous region (membrane) leading to a simulated pressure drop. The simulations demonstrated that each of the design geometries affected the tangential flow across the surface of the membrane, and whether recirculation would occur within a filter housing. Moreover, the simulations demonstrated that these flow regimes were controlled by pressure drop and/or flowrate for a given filter housing design. The flow rate cutoffs between the different flow regimes depend on the geometry of the filter cap. Therefore, if the size or angle of a particular design is changed it is expected that the flow rates defining the different flow regimes will change as well. In many cases, the flow rates are only important to the extent necessary to specify a flow rate for which an intended effect (e.g., recirculation) is achieved for that design.

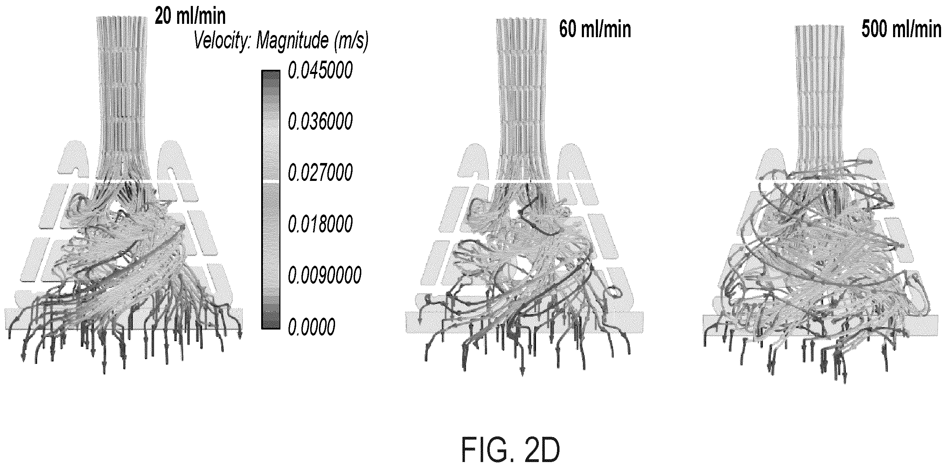

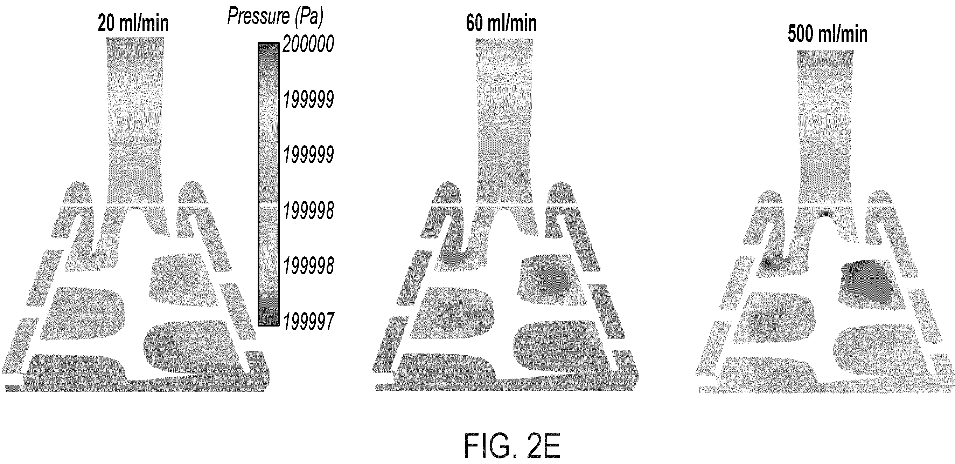

Figs. 2D-2F show the simulated flow within the filter cap 200 of Fig. 2A under increasing flow rate conditions. At flow rates of 20 ml/min, the flow was laminar through the cap and no recirculation is achieved. As flow is increased to 60 ml/min, the flow transitions to turbulent and recirculation begins to occur. At 500 ml/min, significant recirculation is achieved as shown in Fig. 2C. The recirculation flow rate for the filter cap 200 was 82 ml/min, or 16% of the total flow at 500 ml/min. There are several differences in the behavior of the velocity field for the three different flow rates. The rotational flow motion in the inner helix increases with flow rate. The maximum velocity moves from the inlet to the beginning of the inner helix with increased flow. Fig. 2E shows pressure drop and that a low pressure zone appears in the upper part of the helix due to swirling, and once that pressure difference is large enough, recirculation begins to occur. The pressure drop over the filter cap 200 was 650 Pa.

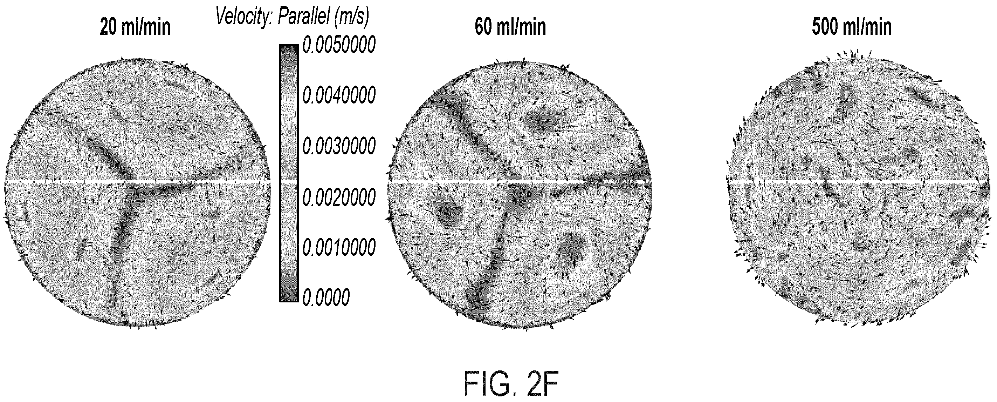

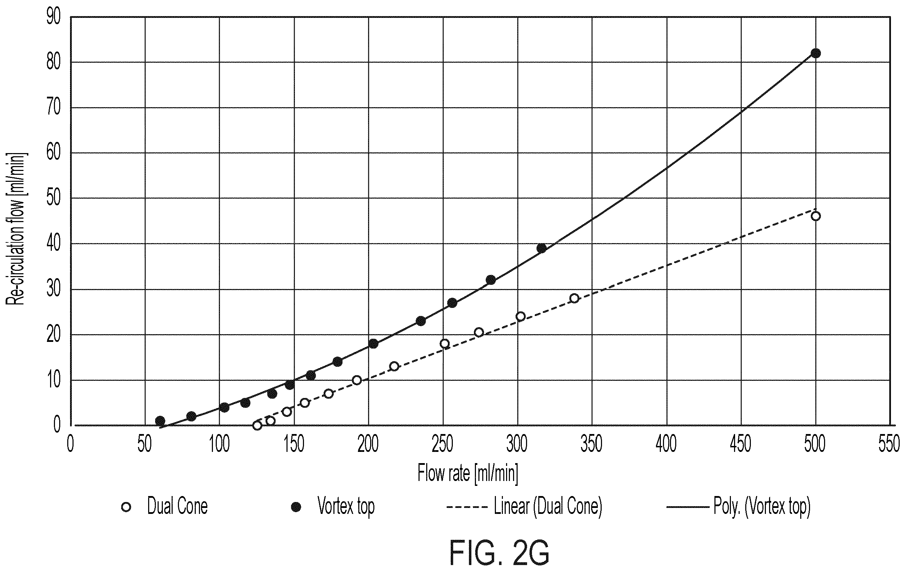

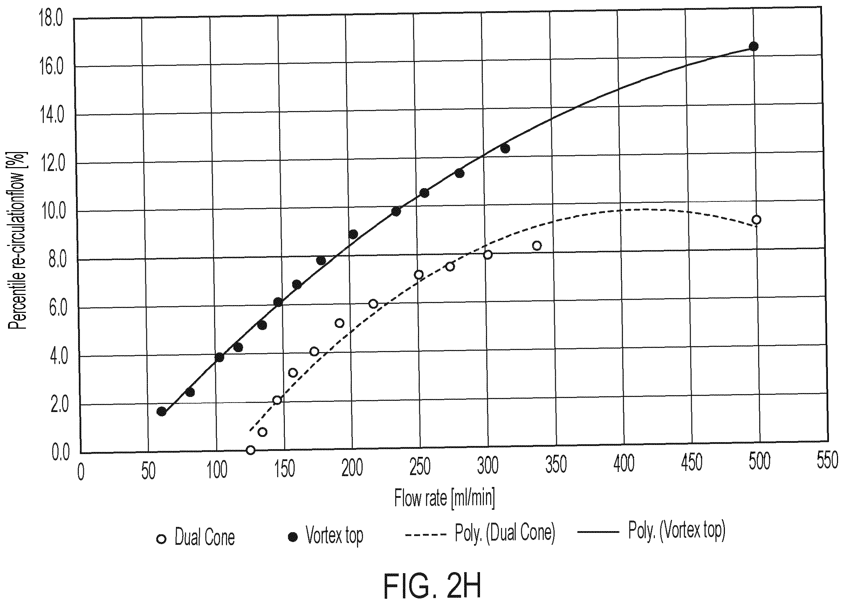

Fig. 2F shows the changing flow rate at 0.25mm above the membrane for each of the flowrates 20ml/min, 60 ml/min, and 500 ml/min. The flow over the membrane changes between the cases. The 20 ml/min shows the pattern resulting from laminar flow, the 60 ml/min case shows a similar pattern as the flow transitions to turbulent. The 500 ml/min case shows chaotic behavior resulting from turbulent flow and recirculation. The recirculation flowrate starts to occur at a flow rate of 60 ml/min for the filter cap 200 (Vortex top) and then increases with flow rate in a quadratic manner as shown in Fig. 2G. The percentage recirculation increases with flow rate as shown in Fig. 2H.

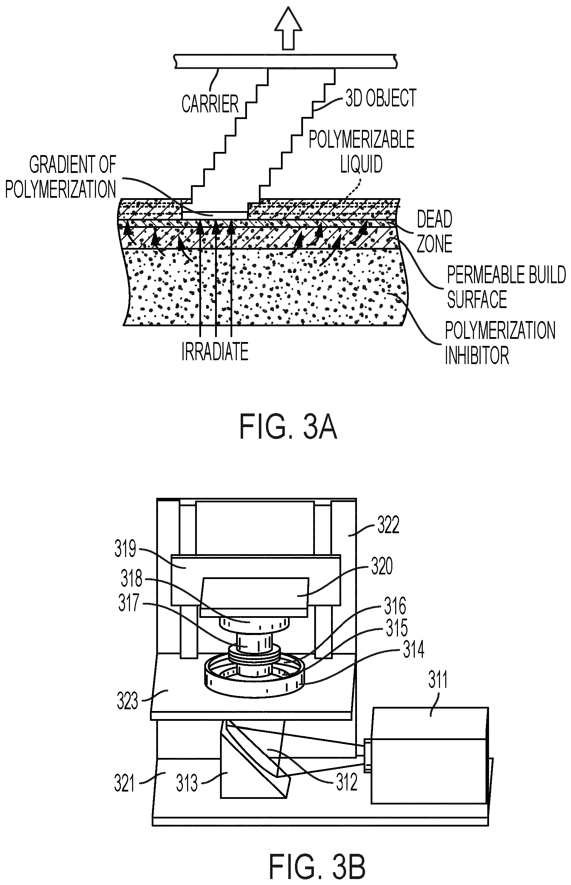

As noted above, the filter housings and filter caps according to examples can generally be manufactured using additive manufacturing processes. One additive manufacturing process that may be used is CLIP, which can be conducted in additive manufacturing machines sold by Carbon3D and described in WO2015/195924, entitled "Three-Dimensional Printing with Reciprocal Feeding of Polymerizable liquid". Fig. 3A shows how materials are build up in the CLIP process, and Fig. 3B shows an apparatus for performing the clips process.

The CLIP process provides a method of forming a three-dimensional object, comprising: (1) providing a carrier 301 and an optically transparent member having a build surface 306, the carrier 301 and the build surface 306 defining a build region therebetween; (2) filling the build region with a polymerizable liquid 304, continuously or intermittently irradiating the build region with light 308 through the optically transparent member to form a solid polymer from the polymerizable liquid 304, and (3) continuously or intermittently advancing ( e.g., sequentially or concurrently with the irradiating step) the carrier 301 away from the build surface to form the three-dimensional object 302 from the solid polymer.

The illumination may be carried out sequentially, and preferably at higher intensity ( e.g., in "strobe" mode). The fabrication may be carried out in two or three sequential patterns, from a base zone, through an optional transition zone, to a body zone, as described further below. The carrier may be vertically reciprocated with respect to the build surface, to enhance or speed the refilling of the build region with the polymerizable liquid. The CLIP process may also involve the filling, irradiating, and/or advancing steps are carried out while also concurrently: (i) continuously maintaining a dead zone of polymerizable liquid in contact with the build surface, and (ii) continuously maintaining a gradient of polymerization zone 303 (which, as discussed below, may also be described as an active surface on the bottom of the growing three dimensional object) between the dead zone and the solid polymer and in contact with each thereof, the gradient of polymerization zone 303 comprising the polymerizable liquid in partially cured form.

Stated differently, in some preferred embodiments of CLIP, the three-dimensional object, or at least some contiguous portion thereof, is formed or produced in situ. "In situ" as used herein has its meaning in the field of chemical engineering, and means "in place." For example, where both the growing portion of the three-dimensional object and the build surface (typically with their intervening active surface or gradient of polymerization 303, and dead zone 305) are maintained in place during formation of at least a portion of the 3D object, or sufficiently in place to avoid the formation of fault lines or planes in the 3D object. For example, in some examples, different portions of the 3D object, which are contiguous with one another in the final 3D object, can both be formed sequentially from or within a gradient of polymerization or active surface 303. Furthermore, a first portion of the 3D object can remain in the gradient of polymerization 303 or contacting the active surface while a second portion, that is contiguous with the first portion, is formed in the gradient of polymerization 303. Accordingly, the 3D object can be remotely fabricated, grown or produced continuously from the gradient of polymerization or active surface (rather than fabricated in discrete layers). The dead zone 305 and gradient of polymerization zone/active surface 303 may be maintained through some or all of the formation of the object being made, for example (and in some embodiments) for a time of at least 5, 10, 20, or 30 seconds, and in some embodiments for a time of at least 1 or 2 minutes.

The apparatus for conducting CLIP is shown in Fig. 3B. It comprises a radiation source 11 such as a digital light processor (DLP) providing electromagnetic radiation 12 which though reflective mirror 13 illuminates a build chamber defined by wall 14 and a rigid build plate 15 forming the bottom of the build chamber, which build chamber is filled with liquid resin 16. The bottom of the chamber 15 is constructed of build plate comprising a semipermeable member as discussed further below. The top of the object under construction 17 is attached to a carrier 18. The carrier is driven in the vertical direction by linear stage 19, although alternate structures can be used as discussed below.

In one aspect, the filter cap shown in Fig. 2A may be made using the CLIP process. In such case the filter cap may be made by building the part layer by layer on the carrier as shown in Fig. 3A. Successive cross sections or build layers define an internal geometry having a helical shape. Because additive manufacturing defines these geometries by successive building layer by layer, the product is a filter cap having an internal structure that could not be made using conventional techniques, such as injection molding or subtractive techniques such as CNC milling. While CLIP is described herein as an advantageous method for making the filter housing components and filter caps according to examples, other additive manufacturing processes described above may be utilized. Of the processes described SLS, SLA, and DLP are particularly well-suited when filter caps according to the examples described herein are made from plastic. Notably, the product may include an additively manufacturer filter cap portion, and conventionally manufactured parts. For example, the base upon which the support mesh and membrane filter are placed may be injection molded. In addition, in the case of a sacrificial mold, the entire part could then be injection molded using a thermoplastic material.

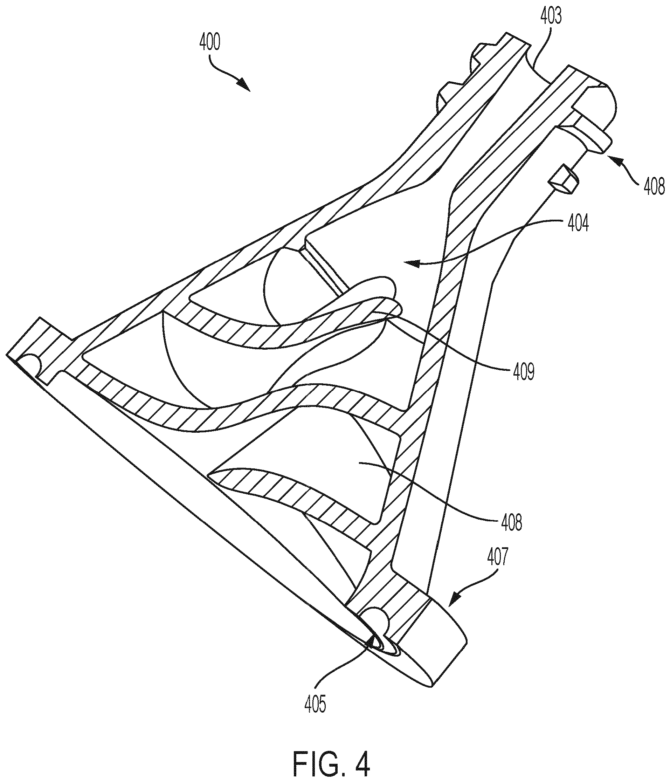

Fig. 4 shows a cap 400 according to an example. This filter cap 400 includes an internal helical structure similar to the filter cap 200 of Fig. 2A, but does not include a recirculation channel. The filter cap 400 is configured to be fitted onto a base (not shown) with a porous membrane (not shown) held between the base and the filter cap. The filter cap 400 includes a groove 405 adapted to accept an O-ring. The filter may have an edge 407 that can be clamped against the base during operation in a conventional manner, using a threaded ring (not shown) that interfaces with a threaded portion of the base (not shown). The filter cap 400 includes an inlet 403. The outer side of the filter cap 400 inlet includes grooves 408, such as luer lock threads that are configured to interface with an inlet tube or syringe. Alternatively, the grooves could be replaced with barbs for holding a tube in place or have a smooth surface. The filter cap 400 includes an internal passage 404 and internal ridges 409 with internal helical passages 408 that can create a vortex flow over the surface of the porous membrane at certain flow conditions.

The internal ridges 409 may be adjusted as desired. If it is desired to increase the residence time of fluid within the cap the angle of the ridges 409 may be decreased such that the fluid travels in a more lateral flow pattern around the circumference of the cap. In some cases, the lower angle of attack may result in a larger number of ridges 409 within the cap. The fluid will emerge over the membrane surface having a greater lateral component of motion in this case. For example, one or more internal ridges 409 may be provided having an angle/angles with respect to a plane perpendicular to a central axis of the filter cap 400 from about 5° or 10° to about 30°, from about 5°, 10°, 15°, etc. to about 10°, 20°, 30°, 40°, 45°. Alternatively, the angle of the ridges 409 may be increased if desired. In this case, the fluid will emerge over the membrane surface having a greater vertical component of motion. For example, one or more internal ridges 409 may be provided having an angle/angles with respect to a plane perpendicular to a central axis of the filter cap 400 from about 45° or 50° to about 75°, from about 45°, 50°, 55°, 60° etc. to about 50°, 60°, 70°, 80°, 85°, 90°. Depending on the application, these factors may be adjusted to enhance recirculation, flow rate, and clogging tendency of the filter.

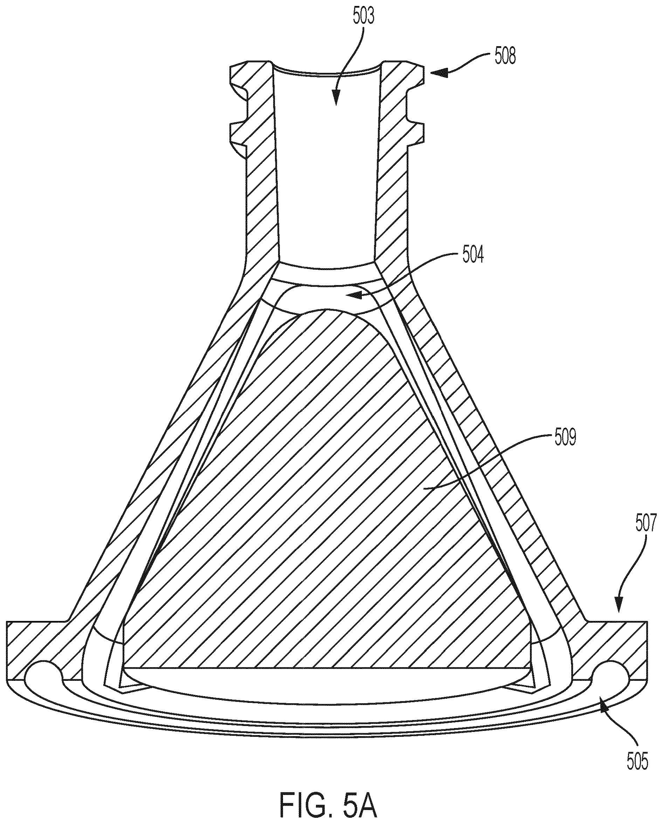

Fig. 5A shows a dual cone filter cap 500 according to an example. The filter cap 500 is configured to be fitted onto a base (not shown) with a porous membrane (not shown) held between the base and the filter cap. The filter cap 500 includes a groove 505 adapted to accept an O-ring. The filter may have an edge 507 that can be clamped against the base during operation in a conventional manner, using a threaded ring (not shown) that interfaces with a threaded portion of the base (not shown). The filter cap 500 includes an inlet 503. The outer side of the filter cap 500 inlet includes grooves 508, such as luer lock threads that are configured to interface with an inlet tube or syringe. Alternatively, the grooves could be replaced with barbs for holding a tube in place or have a smooth surface. The filter cap 500 includes an internal passage 504 and an internal cone 509 that can create a fluid passage 504 for the flow.

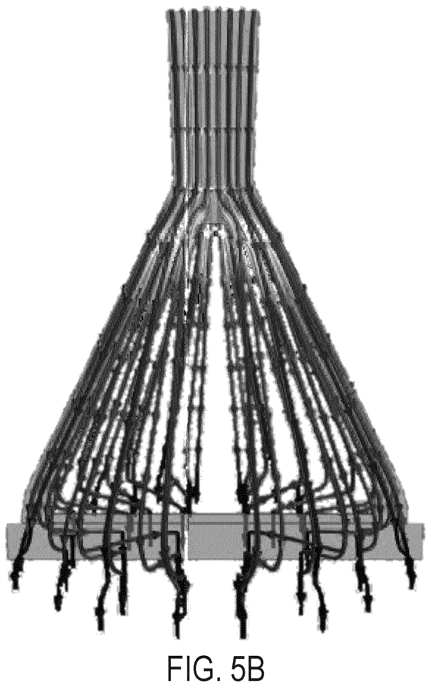

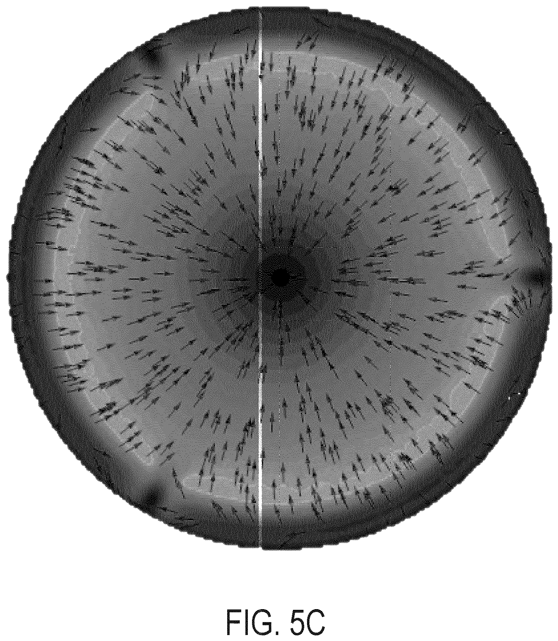

Fig. 5B and 5C depict the simulated flow path within the filter cap 500. The simulated flow path shows tangential flow across the filter membrane, which may be desirable for particular applications. In this case, a pure radial flow across the membrane. This can be compared to the designs giving rise to a rotational flow which increases the flow across the membrane versus through the membrane.

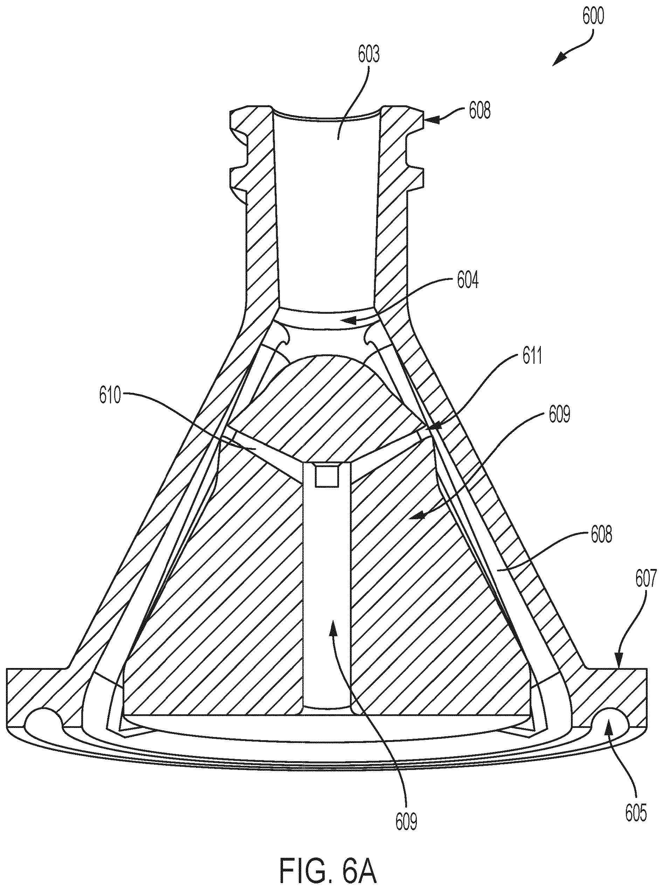

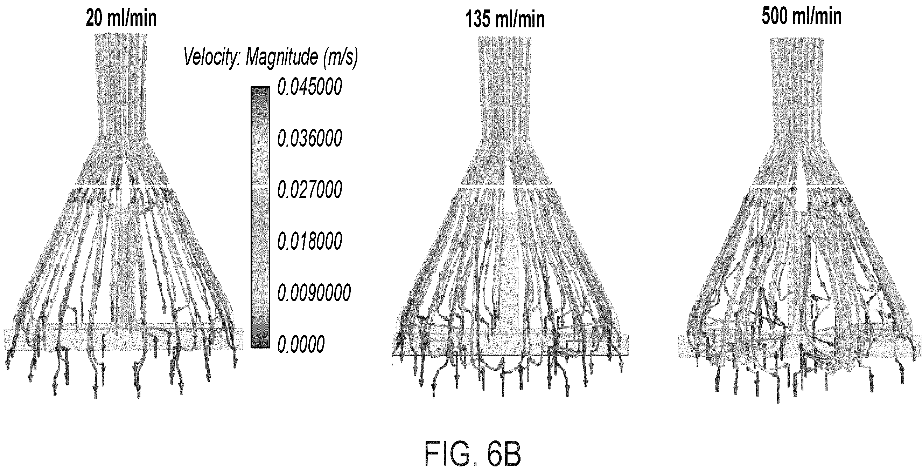

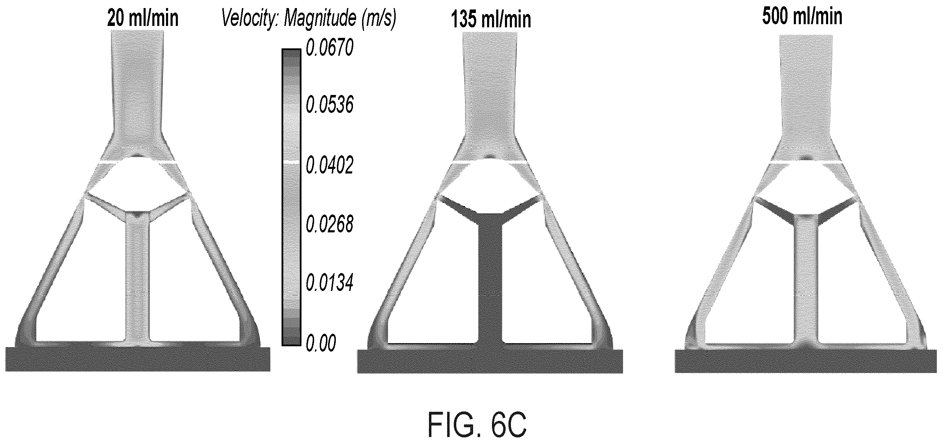

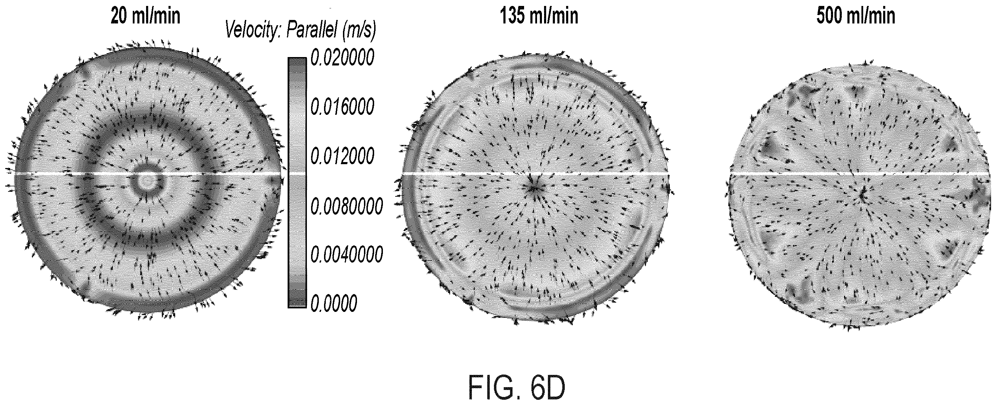

Fig. 6 shows a dual cone recirculating filter cap 600 according to an example. The filter cap 600 is configured to be fitted onto a base (not shown) with a porous membrane (not shown) held between the base and the filter cap. The filter cap 600 includes a groove 605 adapted to accept an O-ring. The filter may have an edge 607 that can be clamped against the base during operation in a conventional manner, using a threaded ring (not shown) that interfaces with a threaded portion of the base (not shown). The filter cap 600 includes an inlet 603. The outer side of the filter cap 600 inlet includes grooves 608, such as luer lock threads that are configured to interface with an inlet tube or syringe. Alternatively, the grooves could be replaced with barbs for holding a tube in place or have a smooth surface.

The filter cap 600 includes an internal inlet passage 604 and an internal cone 609 that can create a fluid passage 608 between the outer wall of the cap for the flow path. The cap 600 also includes a central recirculation channel 609 and upper recirculation channels 610 that provides a flow path for recirculated fluid to enter the internal passage 608. The upper recirculation channels 610 may include a flow restriction feature 611, or venturi contracta, that can modulate recirculated flow through the cap during operation. The channel 610 may be positioned to intersect fluid passage 608 in the center of the flow restriction 611 as shown. Alternatively, the channel 610 may be positioned such that it intersects fluid passage 608 downstream or just after the flow restriction feature 611. This modification would increase the rate of recirculation due to the low pressure zone created by the flow restriction feature.

Figs. 6B-6E show the simulated flow within the filter cap of Fig. 6A under increasing flow rate conditions. The recirculation is illustrated in Fig. 6B. The stream lines does not show the re-circulation flow in the 135 ml/min case (where re-circulation begins) due to the low flow, ~ 1 ml/min in the recirculation channel. As shown in Fig. 6C, the flow is not necessary turbulent at 135 ml/min but the velocity over the venturi contracta is large enough to generate the pressure difference needed to drive the re-circulation. The 135 ml/min and 500 ml/min cases have their maximum velocity over the venturi contracta. This is what creates the pressure drop necessary to drive re-circulation. In the 20 ml/min case the maximum occurs before the venturi contracta. The lowest pressure is observed after the venturi contracta for both the 135 ml/min and 500 ml/min case. That this behavior is seen for the 135 ml/min case suggest that it is due to the venturi contracta and not the re-circulated flow, as the re-circulation is very small (~1 ml/min). It is likely that the lowest pressure will be in the same place even if the re-circulation outlets are moved.

Fig. 6C shows the flow at 0.25 mm above the membrane surface. In contrast to the previous images the flow over the membrane for the 135 ml/min case differs from that of the 500 ml/min case. In fact, it behaves more like the flow in the Dual cone without a re-circulation channel (Fig. 5A, 5C). Because at 135 m/min, the flow is just beginning to transition to turbulent flow there is a large difference between the 135 ml/min and 500 ml/min cases.

The recirculating filter caps of Fig. 2A and 6A provide different flow characteristics that may be desirable for different applications. Recirculation begins at a flow rate of approximately 60 ml/min for the Vortex top and 135 ml/min for the Dual cone. Fig. 2G. The percentile recirculation is higher for the Vortex top than for the Dual cone. Fig. 2H. The percentile re-circulation increases with flow rate for both designs but the increase rate is lower for the Dual cone after about 250 ml/min. Once turbulence is fully developed, further increases in flow rate only costs in pressure loss without further increasing recirculation. Thus, particular designs have optimal flowrates for achieving recirculation while minimizing pressure drop.

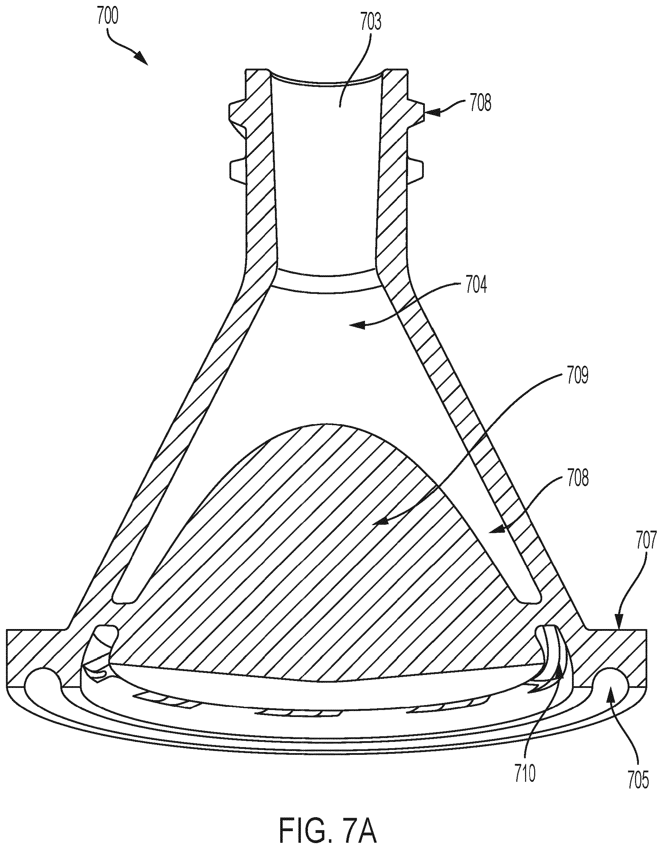

Fig. 7A shows a filter cap 700 according to an example. The filter cap 700 has a dual cone configuration with guide vanes. The filter cap 700 has an inlet passage 704 that is larger than the inlet passage of in the dual cone filter 500 of Fig. 5A. The filter cap 700 is configured to be fitted onto a base (not shown) with a porous membrane (not shown) held between the base and the filter cap. The filter cap 700 includes a groove 705 adapted to accept an O-ring. The filter may have an edge 707 that can be clamped against the base during operation in a conventional manner, using a threaded ring (not shown) that interfaces with a threaded portion of the base (not shown). The filter cap 700 includes an inlet 703. The outer side of the filter cap 700 inlet includes grooves 708 adapted to interface with an inlet tube or syringe. The filter cap 700 includes an inlet passage 704 and an internal cone 709 that can create a fluid passage 708 for the flow path. The filter cap 700 includes spiral guide vanes 710 that cause a spiral flow in the fluid leaving the fluid passage 708 over the surface of the porous membrane. This flow pattern increases the amount of fluid traveling tangentially over the membrane surface relative to fluid directly passing through the membrane.

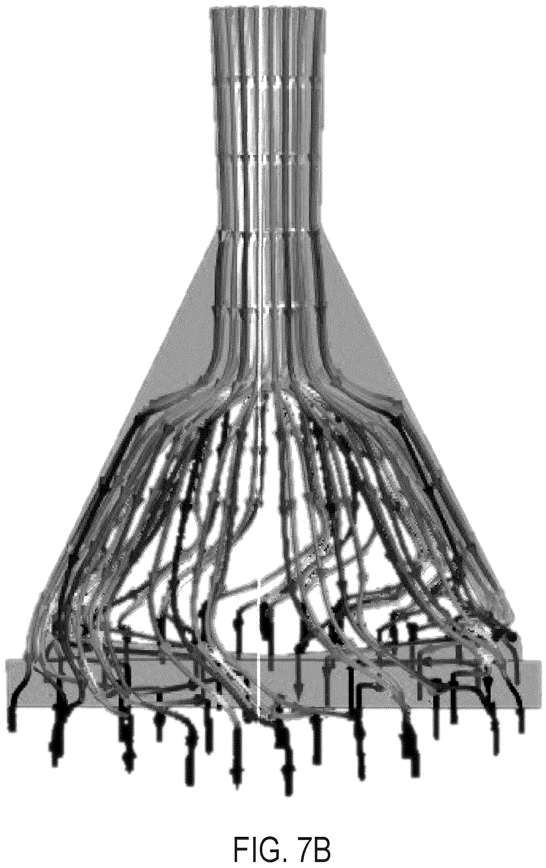

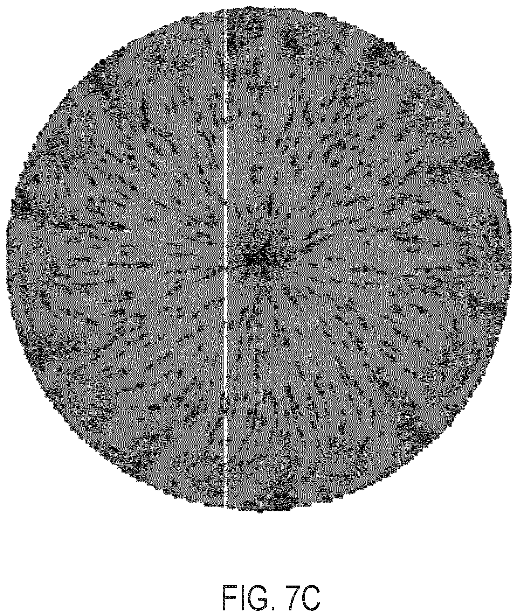

Fig. 7B and 7C depict the simulated flow path within the filter cap 700. The simulated flow path shows spiral flow over the filter membrane, which may be desirable for particular applications.

Other embodiments and uses of the invention will be apparent to those skilled in the art from consideration of the specification and practice of the invention disclosed herein. It is intended that the specification and examples be considered exemplary only, with the scope of the invention being indicated by the following claims.

## Claims

1. A filter cap (200, 400, 500, 600, 700) for holding a filter membrane against a base in an inline filter assembly, where fluid passes from the filter cap (200, 400, 500, 600, 700) through the filter membrane and out of the base, the filter cap (200, 400, 500, 600, 700) being for bioprocessing and comprising:
a conical shaped portion, where the conical shape is configured to distribute fluid over a surface of a filter membrane; and
at least one flow conduit (204, 404, 504, 608, 708) within the conical shape portion, wherein the flow conduit (204, 404, 504, 608, 708) includes a vortex feature (209, 409, 509, 609, 709) configured to provide vortex flow within the filter cap.

2. The filter cap (200, 400, 500, 600, 700) of claim 1, wherein the flow conduit (204, 404, 504, 608, 708) is further capable of providing at least one of recirculating flow, spiral flow, and/or cross-flow across the membrane.

3. The filter cap (200, 400, 500, 600, 700) of claim 1 or 2, wherein the vortex feature (209, 409, 509, 609, 709) comprises one or more internal ridges 409 having an angle/angles with respect to a plane perpendicular to a central axis of the filter cap 400 from about 5° or 10° to about 30°, and/or from about 5°, 10°, 15°, 25° to about 10°, 20°, 30°, 40°, 45° and/or from about 45° or 50° to about 75°, and/or from about 45°, 50°, 55°, 60°, 70°, 80° to about 50°, 60°, 70°, 80°, 85°, 90°.

4. The filter (200, 400, 500, 600, 700) cap of any preceding claim, wherein the vortex feature (209, 409, 509, 609, 709) is and/or provides a helical structure (408).

5. The filter cap (200, 400, 500, 600, 700) of claim 4, wherein the flow conduit (204, 404, 504, 608, 708) includes a recirculating structure, wherein the recirculating structure comprises a channel (202, 609, 610) located radially outward from the helical structure (408).

6. The filter cap (200, 400, 500, 600, 700) of any preceding claim, wherein the flow conduit (204, 404, 504, 608, 708) is capable of providing cross-flow across the membrane, and cross-flow increases the flow across the surface of the membrane relative to flow through the membrane.

7. The filter cap (200, 400, 500, 600, 700) of any preceding claim, wherein the flow conduit (204, 404, 504, 608, 708) includes a recirculating conduit (608).

8. The filter cap (200, 400, 500, 600, 700) of claim 7, wherein the recirculating conduit (608) includes a flow restriction feature (611).

9. The filter cap (200, 400, 500, 600, 700) of claim 8, wherein the flow restriction feature (611) is a venturi contraction.

10. The filter cap (200, 400, 500, 600, 700) of any preceding claim, wherein the filter cap includes spiral-shaped guide vanes (710).

11. A method of making the filter cap (200, 400, 500, 600, 700) of any preceding claim, wherein the method includes at least one additive manufacturing step.

12. The method of claim 11, wherein the additive manufacturing comprises selective laser sintering (SLS), stereolithography (SLA), digital light projection (DLP), continuous liquid interface programming (CLIP), binder jetting, selective hot sintering (SHS), fused deposition modeling (FDM), direct metal laser sintering (DMLS) or directed energy deposition (DED).

13. The method of claim 11 or 12, wherein the additive manufacturing comprises selective laser sintering (SLS), stereolithography (SLA), digital light projection (DLP), continuous liquid interface programming (CLIP), binder jetting, selective hot sintering (SHS)

14. A method of filtering a using the filter cap (200, 400, 500, 600, 700) of any of claims 1 to 10.

15. The method of claim 14, wherein the filtering comprises filtering a biological product, optionally wherein the biological product is a single use, sterile biological product.

16. The method of claims 14 or 15, wherein: i) the filter cap (200, 400, 500, 600, 700) is used in a filter assembly, and the flow rate is at least 60 ml/min; ii) wherein the filtering results in recirculation that allows prolonged use of a filter media compared to a filtering without recirculation; iii) wherein the flowrate is at a level that provides recirculation within the filter cap (200, 400, 500, 600, 700); iv) wherein the flowrate is at least 500 ml/min; and/or v) wherein the flowrate is at least 1000 ml/min.

## Patentansprüche

1. Filterkappe (200, 400, 500, 600, 700) zum Halten einer Filtermembran gegen eine Basis in einer Inline-Filteranordnung, wobei Fluid von der Filterkappe (200, 400, 500, 600, 700) durch die Filtermembran und dann aus der Basis fließt, wobei die Filterkappe (200, 400, 500, 600, 700) für die Bioverarbeitung ist und Folgendes umfasst:
einen konisch geformten Abschnitt, wobei die konische Form so konfiguriert ist, dass sie Fluid über eine Oberfläche einer Filtermembran verteilt; und
mindestens eine Strömungsleitung (204, 404, 504, 608, 708) innerhalb des konischen Abschnitts, wobei die Strömungsleitung (204, 404, 504, 608, 708) eine Wirbeleinrichtung (209, 409, 509, 609, 709) einschließt, die so konfiguriert ist, dass sie eine Wirbelströmung innerhalb der Filterkappe bereitstellt.

2. Filterkappe (200, 400, 500, 600, 700) nach Anspruch 1, wobei die Strömungsleitung (204, 404, 504, 608, 708) weiter in der Lage ist, mindestens eines von Rückführungsströmung, Spiralströmung und/oder Querströmung über die Membran bereitzustellen.

3. Filterkappe (200, 400, 500, 600, 700) nach Anspruch 1 oder 2, wobei die Wirbeleinrichtung (209, 409, 509, 609, 709) eine oder mehrere innere Rippen 409 umfasst, die einen bzw. mehrere Winkel in Bezug auf eine Ebene senkrecht zu einer Mittelachse der Filterkappe 400 von etwa 5° oder 10° bis etwa 30° aufweisen, und/oder von etwa 5°, 10°, 15°, 25° bis etwa 10°, 20°, 30°, 40°, 45° und/oder von etwa 45° oder 50° bis etwa 75°, und/oder von etwa 45°, 50°, 55°, 60°, 70°, 80° bis etwa 50°, 60°, 70°, 80°, 85°, 90°.

4. Filterkappe (200, 400, 500, 600, 700) nach einem der vorstehenden Ansprüche, wobei die Wirbeleinrichtung (209, 409, 509, 609, 709) eine spiralförmige Struktur (408) ist und/oder bereitstellt.

5. Filterkappe (200, 400, 500, 600, 700) nach Anspruch 4, wobei die Strömungsleitung (204, 404, 504, 608, 708) eine Rückführungsstruktur einschließt, wobei die Rückführungsstruktur einen Kanal (202, 609, 610) umfasst, der radial außerhalb der spiralförmigen Struktur (408) angeordnet ist.

6. Filterkappe (200, 400, 500, 600, 700) nach einem der vorstehenden Ansprüche, wobei die Strömungsleitung (204, 404, 504, 608, 708) in der Lage ist, eine Querströmung über die Membran bereitzustellen, und die Querströmung die Strömung über die Oberfläche der Membran im Verhältnis zur Strömung durch die Membran erhöht.

7. Filterkappe (200, 400, 500, 600, 700) nach einem der vorstehenden Ansprüche, wobei die Strömungsleitung (204, 404, 504, 608, 708) eine Rückführungsleitung (608) einschließt.

8. Filterkappe (200, 400, 500, 600, 700) nach Anspruch 7, wobei die Rückführungsleitung (608) eine Durchflussbegrenzungseinrichtung (611) einschließt.

9. Filterkappe (200, 400, 500, 600, 700) nach Anspruch 8, wobei die Strömungsbegrenzungseinrichtung (611) eine Venturi-Verengung ist.

10. Filterkappe (200, 400, 500, 600, 700) nach einem der vorstehenden Ansprüche, wobei die Filterkappe spiralförmige Leitschaufeln (710) einschließt.

11. Verfahren zur Herstellung der Filterkappe (200, 400, 500, 600, 700) nach einem der vorstehenden Ansprüche, wobei das Verfahren mindestens einen additiven Fertigungsschritt einschließt.

12. Verfahren nach Anspruch 11, wobei die additive Fertigung selektives Lasersintern (SLS), Stereolithografie (SLA), digitale Lichtprojektion (DLP), Continuous Liquid Interface Programming (CLIP), Binder Jetting, selektives Heißsintern (SHS), Fused Deposition Modeling (FDM), direktes Metall-Lasersintern (DMLS) oder gerichtete Energieablage (DED) umfasst.

13. Verfahren nach Anspruch 11 oder 12, wobei die additive Fertigung selektives Lasersintern (SLS), Stereolithografie (SLA), digitale Lichtprojektion (DLP), Continuous Liquid Interface Programming (CLIP), Binder Jetting und selektives Heißsintern (SHS) umfasst.

14. Verfahren zum Filtern unter Verwendung der Filterkappe (200, 400, 500, 600, 700) nach einem der Ansprüche 1 bis 10.

15. Verfahren nach Anspruch 14, wobei das Filtern das Filtern eines biologischen Produkts umfasst, optional wobei das biologische Produkt ein steriles biologisches Produkt zum einmaligen Gebrauch ist.

16. Verfahren nach den Ansprüchen 14 oder 15, wobei: i) die Filterkappe (200, 400, 500, 600, 700) in einer Filteranordnung verwendet wird und die Strömungsgeschwindigkeit mindestens 60 ml/min beträgt; ii) wobei das Filtern zu einer Rückführung führt, die eine längere Verwendung eines Filtermediums im Vergleich zu einem Filtern ohne Rückführung ermöglicht; iii) wobei die Strömungsgeschwindigkeit auf einem Niveau liegt, das eine Rückführung innerhalb der Filterkappe (200, 400, 500, 600, 700) ermöglicht; iv) wobei die Strömungsgeschwindigkeit mindestens 500 ml/min beträgt; und/oder v) wobei die Strömungsgeschwindigkeit mindestens 1000 ml/min beträgt.

## Revendications

1. Capuchon (200, 400, 500, 600, 700) de filtre pour maintenir une membrane filtrante contre une base dans un ensemble filtre en ligne, dans lequel un fluide passe depuis le capuchon (200, 400, 500, 600, 700) de filtre à travers la membrane filtrante et hors de la base, le capuchon (200, 400, 500, 600, 700) de filtre étant destiné au biotraitement et comprenant :
une partie de forme conique, dans laquelle la forme conique est configurée pour distribuer un fluide sur une surface d'une membrane filtrante ; et
au moins un conduit (204, 404, 504, 608, 708) d'écoulement à l'intérieur de la partie de forme conique, dans lequel le conduit (204, 404, 504, 608, 708) d'écoulement inclut un élément de tourbillon (209, 409, 509, 609, 709) configuré pour fournir un écoulement tourbillonnaire à l'intérieur du capuchon de filtre.

2. Capuchon (200, 400, 500, 600, 700) de filtre selon la revendication 1, dans lequel le conduit (204, 404, 504, 608, 708) d'écoulement est en outre capable de fournir au moins un parmi un écoulement en recirculation, un écoulement en spirale et/ou un écoulement transversal à travers la membrane.

3. Capuchon (200, 400, 500, 600, 700) de filtre selon la revendication 1 ou la revendication 2, dans lequel l'élément de tourbillon (209, 409, 509, 609, 709) comprend une ou plusieurs arêtes internes 409 présentant un/des angle(s) par rapport à un plan perpendiculaire à un axe central du capuchon 400 de filtre d'environ 5° ou 10° à environ 30°, et/ou d'environ 5°, 10°, 15°, 25° à environ 10°, 20°, 30°, 40°, 45° et/ou d'environ 45° ou 50° à environ 75°, et/ou d'environ 45°, 50°, 55°, 60°, 70°, 80° à environ 50°, 60°, 70°, 80°, 85°, 90°.

4. Capuchon (200, 400, 500, 600, 700) de filtre selon une quelconque revendication précédente, dans lequel l'élément de tourbillon (209, 409, 509, 609, 709) est et/ou fournit une structure hélicoïdale (408).

5. Capuchon (200, 400, 500, 600, 700) de filtre selon la revendication 4, dans lequel le conduit (204, 404, 504, 608, 708) d'écoulement inclut une structure de recirculation, dans lequel la structure de recirculation comprend un canal (202, 609, 610) situé radialement vers l'extérieur à partir de la structure hélicoïdale (408).

6. Capuchon (200, 400, 500, 600, 700) de filtre selon une quelconque revendication précédente, dans lequel le conduit (204, 404, 504, 608, 708) d'écoulement est capable de fournir un écoulement transversal à travers la membrane, et l'écoulement transversal augmente l'écoulement à travers la surface de la membrane par rapport à l'écoulement à travers la membrane.

7. Capuchon (200, 400, 500, 600, 700) de filtre selon une quelconque revendication précédente, dans lequel le conduit (204, 404, 504, 608, 708) d'écoulement inclut un conduit (608) de recirculation.

8. Capuchon (200, 400, 500, 600, 700) de filtre selon la revendication 7, dans lequel le conduit (608) de recirculation inclut un élément (611) de restriction d'écoulement.

9. Capuchon (200, 400, 500, 600, 700) de filtre selon la revendication 8, dans lequel l'élément (611) de restriction d'écoulement est une contraction venturi.

10. Capuchon (200, 400, 500, 600, 700) de filtre selon une quelconque revendication précédente, dans lequel le capuchon de filtre inclut des aubes de guidage (710) en forme de spirale.

11. Procédé de fabrication du capuchon (200, 400, 500, 600, 700) de filtre selon une quelconque revendication précédente, dans lequel le procédé inclut au moins une étape de fabrication additive.

12. Procédé selon la revendication 11, dans lequel la fabrication additive comprend le frittage laser sélectif (SLS), la stéréolithographie (SLA), la projection de lumière numérique (DLP), la programmation d'interface liquide continue (CLIP), le jet de liant, le frittage à chaud sélectif (SHS), la modélisation par dépôt fusionné (FDM), le frittage laser direct sur métal (DMLS) ou le dépôt à énergie dirigée (DED).

13. Procédé selon la revendication 11 ou la revendication 12, dans lequel la fabrication additive comprend le frittage laser sélectif (SLS), la stéréolithographie (SLA), la projection de lumière numérique (DLP), la programmation d'interface liquide continue (CLIP), le jet de liant, le frittage à chaud sélectif (SHS).

14. Procédé de filtration à l'aide du capuchon (200, 400, 500, 600, 700) de filtre selon l'une quelconque des revendications 1 à 10.

15. Procédé selon la revendication 14, dans lequel la filtration comprend la filtration d'un produit biologique, facultativement dans lequel le produit biologique est un produit biologique stérile à usage unique.

16. Procédé selon la revendication 14 ou la revendication 15, dans lequel : i) le capuchon (200, 400, 500, 600, 700) de filtre est utilisé dans un ensemble filtre, et le débit est d'au moins 60 ml/min ; ii) dans lequel la filtration entraîne une recirculation qui permet une utilisation prolongée d'un matériau filtrant par rapport à une filtration sans recirculation ; iii) dans lequel le débit est à un niveau qui fournit une recirculation à l'intérieur du capuchon (200, 400, 500, 600, 700) de filtre ; iv) dans lequel le débit est d'au moins 500 ml/min ; et/ou v) dans lequel le débit est d'au moins 1000 ml/min.
